# EUROPEAN PATENT SPECIFICATION

(11) **EP 3 908 225 B1**
(45) Date of publication and mention of the grant of the patent: **13.11.2024**
(21) Application number: 20730098.9
(22) Date of filing: 10.01.2020
(51) Int. Cl.: A61B 90/10, A61B 90/14

(54) **ROCKER ARM ASSEMBLY FOR HEAD FIXATION DEVICE**
KIPPHEBELANORDNUNG FÜR EINE KOPFHALTERUNGSVORRICHTUNG
ENSEMBLE CULBUTEUR POUR DISPOSITIF DE FIXATION DE TÊTE

(30) Priority: 10.01.2019 US 201962790785 P; 31.01.2019 US 201962799425 P
(43) Date of publication of application: 17.11.2021
(73) Proprietor: Pro Med Instruments GmbH, 79111 Freiburg im Breisgau (DE)
(72) Inventor: SCHUELE, Matthias, E., 79111 Freiburg (DE); MERTENS, Jan, H., 79115 Freiburg (DE)
(74) Representative: Samson & Partner Patentanwälte mbB
(86) International application number: PCT/IB2020/000022
(87) International publication number: WO 2020/144537

(56) References cited:
- US-A1- 2008 251 086
- US-A1- 2008 251 086
- US-A1- 2010 059 064
- US-A1- 2013 096 557
- US-A1- 2013 096 557
- US-B1- 7 730 563
- US-B1- 7 730 563

## Description

### BACKGROUND

Relevant prior art is disclosed in US 2013/096557, US 7 730 563, US 2008/251086 and US 2010/059064. The devices and unclaimed methods disclosed pertain to patient stabilization, and in particular head and neck stabilization using stabilization devices known as head stabilization devices, which are also referred to as head fixation devices (hereinafter referred to as "HFDs" or "HFD" in singular). HFDs are sometimes used during a variety of surgical and other medical procedures, for example during head or neck surgery or testing where it would be desirable to securely hold a patient's head in a certain position. HFDs can include various components that are configured to contact the head of the patient. Some such components include skull pins or pads that can be retained in a pin or pad holder assembly. Some devices and unclaimed methods disclosed herein pertain to various pin or pad holder assemblies. While a variety of stabilization devices and components thereof have been made and used, it is believed that no one prior to the inventor(s) has made or used an invention as described herein and limited by the scope of appended independent claim 1. Further embodiments are disclosed in the dependent claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements.
FIG. 1 depicts a perspective view of an exemplary HFD in the form of a skull clamp and having an exemplary pin holder assembly configured as a curved 2-pin rocker arm assembly on one side.
FIG. 2 depicts a front cross-sectional view of the curved arm of the 2-pin rocker arm assembly of FIG. 1.
FIG. 3 depicts a front schematic view of the HFD of FIG. 1 showing a stabilization configuration used with an elliptically shaped patient head, showing exemplary penetration angles of a 3-pin fixation.
FIG. 4 depicts a partial enlarged perspective view of an exemplary stabilization configuration, showing a single pin and its penetration angle.
FIG. 5 depicts a partial front view of an exemplary HFD stabilization configuration showing exemplary penetration angles defined by two pins of a curved 2-pin rocker arm assembly when used with a planar portion of cranial bone of the patient's head.
FIG. 6 depicts a partial front view of an exemplary HFD stabilization configuration showing exemplary penetration angles defined by two pins of another exemplary 2-pin rocker arm assembly when used with a planar portion of cranial bone of the patient's head.
FIG. 7 depicts a partial front view in cross section of the HFD stabilization configuration of FIG. 5, shown with the skull pins omitted illustrating exemplary penetration angles defined by two bores of a curved 2-pin rocker arm assembly when used with a planar portion of cranial bone of the patient's head.
FIG. 8 depicts a partial front view in cross section of the HFD stabilization configuration of FIG. 6, shown with the skill pins omitted illustrating exemplary penetration angles defined by two bores when used with a planar portion of cranial bone of the patient's head.
FIG. 9 depicts a perspective view of an exemplary HFD in the form of a skull clamp and having an exemplary pin holder assembly configured with a 2-pin rocker arm assembly on one side configured for contact with planar regions of a patient's head.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

### I. Exemplary Rocker Arm Assemblies Configured for Planar Region Contact

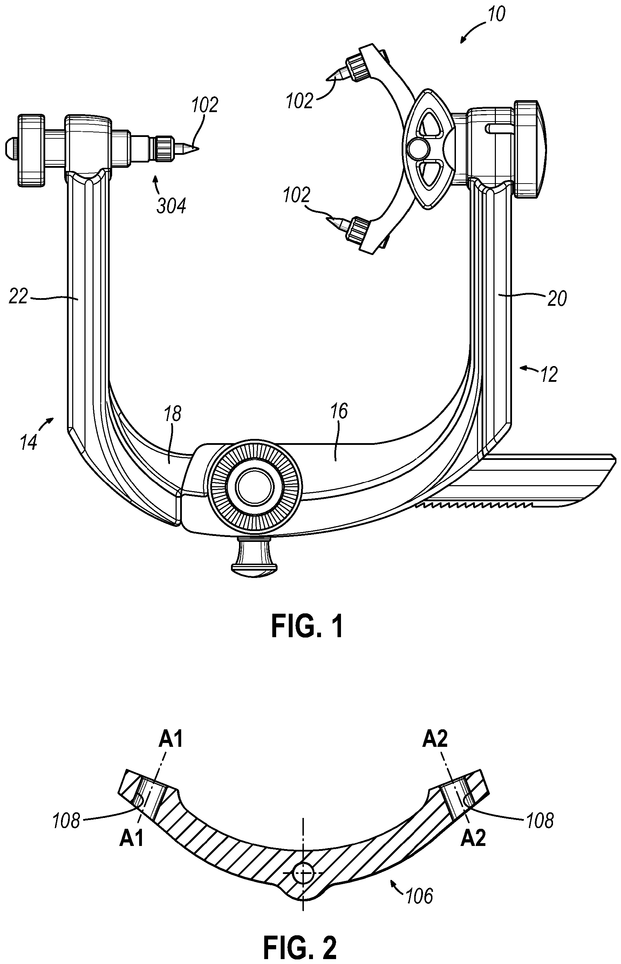

FIG. 1 shows an exemplary HFD in the form of a skull clamp (10) that uses 3-point fixation. Skull clamp (10) comprises a pair of arms (12, 14) with each arm (12, 14) having a lateral portion (16, 18) and an upright portion (20, 22). Arms (12, 14) are adjustable laterally relative to each other so skull clamp (10) accommodates various patient head sizes. Skull clamp (10) includes a rocker arm assembly (100) that holds two pins (102) on one side of skull clamp (10). In the illustrated version, rocker arm assembly (100) is connected with skull clamp (10) at an end of upright portion (20). Opposite rocker arm assembly (100) at an end of upright portion (22) is a single pin holder (104) holding a single pin (102).

FIG. 2 shows an arm (106) of rocker arm assembly (100) in cross section. Arm (106) comprises a curved shaped in the illustrated version, and a pair of bores (108) within arm (106). In this manner, bores (108) are configured to retain pins (102) at each respective end of the arm (106). Arm (106) further angles inward such that longitudinal axes (A1, A2) defined by bores (108) of arm (106) extend in a converging manner as shown.

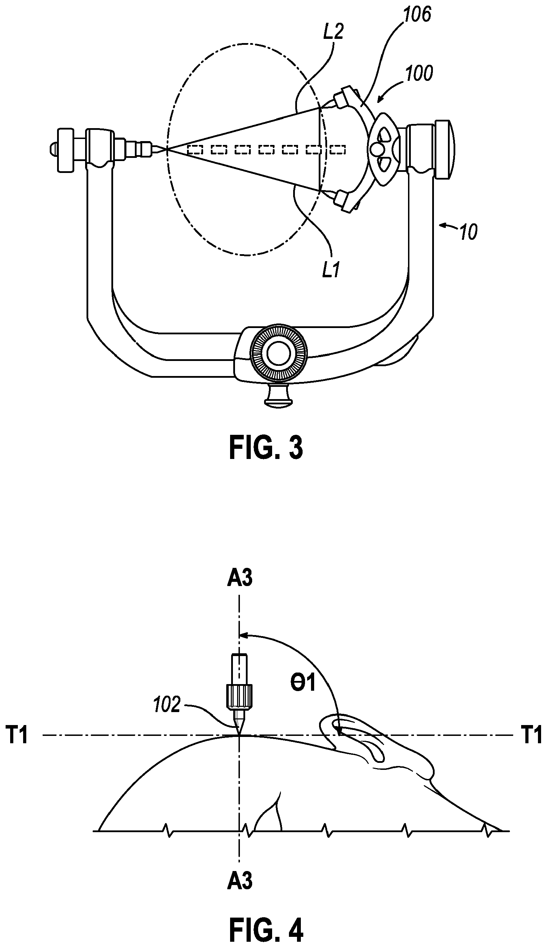

FIG. 3 shows a stabilization arrangement with a head of a patient that is elliptically shaped using skull clamp (10). In the illustrated version, the contact angles defined by the pins and their interface with the patient's head are shown by diagonally extending lines (L1, L2) that form a triangular shape. The term "contact angle" as used herein should be understood to also include the penetration angle when the stabilization involves the pin or contact feature penetrating the bone as opposed to only contacting the bone without penetration. Thus, the term "contact angle" is understood to be inclusive of both the angle of contact and the angle of penetration depending on the configuration of the stabilization. In this example, arm (106) of a rocker arm assembly (100) is configured with a curved shape designed to work with an elliptically shaped head of a patient to provide for a desired contact angle. In some examples where the patient's skull is elliptically shaped, or in other words where the head shape resembles an ellipsoid, the position of all three pins form an isosceles triangle. This provides load distributing among all three pins and the pins are configured with contact angles of ninety or about ninety degrees. In other words, the pins contact and may be driven into the cranial bone at ninety or about ninety degrees or perpendicular or substantially perpendicular to the cranial bone.

FIG. 4 shows a stabilization arrangement where skull pin (102) defines a longitudinal axis (A3), and skull pin (102) is positioned in contact with an elliptically shaped portion of the head of a patient. A tangent line (T1) is shown at the point of contact where skull pin (102) contacts the head. The contact angle (θ1), defined as the angle between longitudinal axis (A3) of skull pin (102) and tangent line (T1), is shown as ninety or about ninety degrees. In this perpendicular or substantially perpendicular arrangement, skull pin (102) makes a rigid and stable connection with the head.

In certain neurosurgical procedures, the patient's skull is pinned in a planar region of the cranial bone rather than an elliptical region as discussed above. The planar region of the cranial bone can be planar, generally planar, or substantially planar over some distance of the bone structure. For the avoidance of doubt, the term "planar region" as used herein should be understood to encompass not only a planar region, but also a generally planar region, substantially planar region, a quasi-planar region, and/or other variations of these terms as will be understood by those of ordinary skill in the art in view of the teachings herein. By way of example only, and not limitation, pinning in a planar region of the cranial bone can be the case in procedures in which the patient is positioned face down (prone position). For instance, in order to perform a surgery in the occipital region, in the posterior fossa, or at the cervical spine, in at least some instances the pins are placed in a planar region of temporal bone and parietal bone.

If using the rocker arm assembly and pins described above, when pinning into a planar region of cranial bone, the contact angle of the pins will differ compared to using that same rocker arm assembly and pins when pinning into an elliptical region of cranial bone. Thus, trying to achieve an about ninety-degree contact angle for the pins would not be attainable when pinning in these different bone geometries using identical rocker arm assemblies and pins. For instance, if pinning using a rocker arm assembly and pins configured for use with an elliptically shaped head to provide for ninety degree contact angle of the pins, such as rocker arm assembly (100) having pins (102) as shown in FIGS. 1-4, using the same rocker arm assembly and pins with a planar region of cranial bone can impede achieving a contact angle of ninety degrees or thereabouts. The result here can be a loss in stability and rigidity of the stabilization.

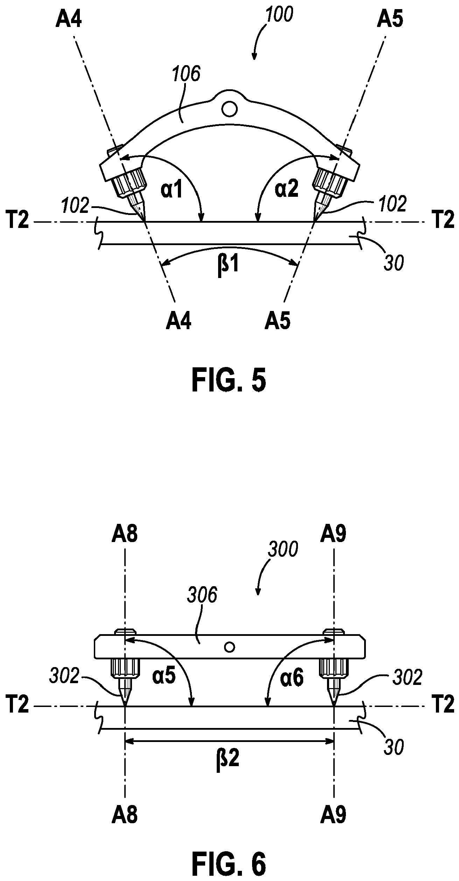

By way of example, FIG. 5 shows portions of rocker arm assembly (100) of FIGS. 1-3 used with a planar portion of cranial bone (30) of the head of the patient. In this example, when pinning into a planar region of cranial bone as shown in FIG. 5, the angle of contact is greater than ninety degrees. More specifically, the contact angles (α1, α2) are defined as the angle between longitudinal axes (A4, A5) of respective skull pins (102) and tangent line (T2).

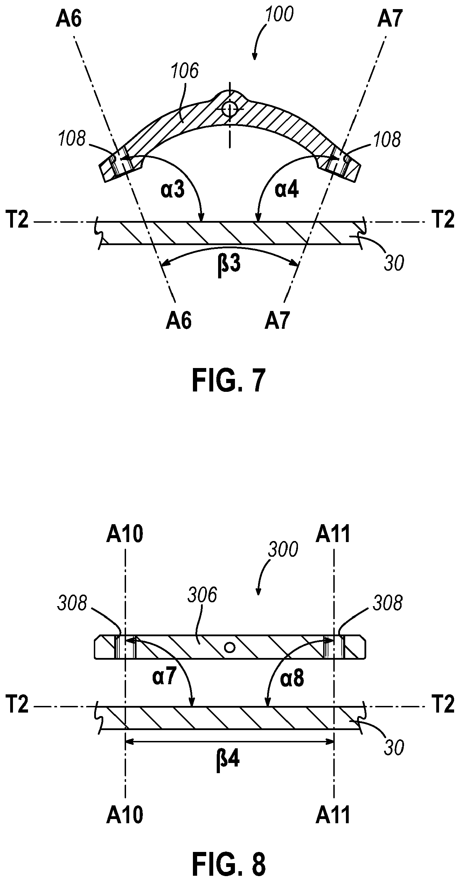

By way of further example, FIG. 7 shows portions of rocker arm assembly (100) of FIG. 5 without pins (102), and again being used with a planar portion of cranial bone (30) of the head of the patient. In this example, when pinning into a planar region of cranial bone as shown in FIG. 7, the angle of contact is greater than ninety degrees. More specifically, the contact angles (α3, α4) are defined as the angle between longitudinal axes (A6, A7) of respective bores (108) and tangent line (T2).

As described above with respect to FIGS. 5 and 7, contact angle can be defined based on the orientation of bores (108) or the orientation of pins (102). In some instances where pins (102) are straight and bores (108) define concentric receptacles for receiving pins (102), the longitudinal axes defined by bores (108) and pins (102) will be the same. In this instance, the contact angles will be the same whether defined by the orientation of bores (108) or the orientation of pins (102). However, in some other examples, bores (108) may be omitted where pins (102) may be formed with or as part of arm (106). In such an example, the contact angle will be defined based on the orientation of the pins (102). Still in some other examples, bores (108) and pins (102) can be shaped or configured such that they do not have the same or a common longitudinal axis, and thus would define different contact angles as defined herein. By way of example only, and not limitation, this could be the case where pins (102) are angled or curved instead of straight. In view of the teachings herein, other configurations for rocker arm assembly (100), with pins (102) and bores (108), where pins (102) and bores (108) have different longitudinal axes, and thus different contact angles as defined herein, will be apparent to those of ordinary skill. In application, where the contact angle defined by the bores and the contact angle defines by the pins differs, the contact angle as defined by the contact features or pins is a key factor when considering the stability of a patient stabilization as it is those contact features or pins that contact the patient's head.

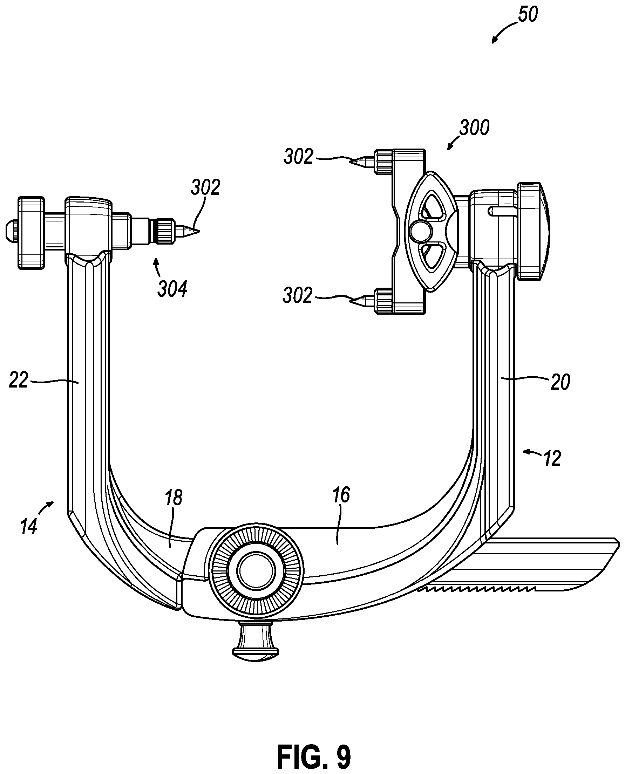

FIG. 9 shows an exemplary HFD in the form of a skull clamp (50) that uses 3-point fixation. Skull clamp (50) comprises pair of arms (12, 14) with each arm (12, 14) having lateral portion (16, 18) and upright portion (20, 22). Arms (12, 14) are adjustable laterally relative to each other so skull clamp (50) accommodates various patient head sizes. Skull clamp (50) includes a rocker arm assembly (300) that holds two pins (302) on one side of skull clamp (50). In the illustrated version, rocker arm assembly (300) is connected with skull clamp (50) at end of upright portion (20). Opposite rocker arm assembly (300) at end of upright portion (22) is a single pin holder (304) holding a single pin (302).

Turning now to FIGS. 6 and 8, FIG. 6 shows portions of exemplary 2-pin rocker arm assembly (300) that is configured for use with skull clamp (50) like shown in FIG. 9, but which also may be configured for use in place of rocker arm assembly (100) of skull clamp (10) as shown in FIGS. 1-3, as well as other types of HFDs. In the illustrated version of FIG. 6, 2-pin rocker arm assembly (300) is used with a planar portion of cranial bone (30) of the head of the patient. In this example, the angle of contact is ninety degrees or about ninety degrees. This is the case for both pins (302) configured to be used with the 2-pin rocker arm assembly (300). More specifically, the contact angles (α5, α6) on each side of arm (306) are defined as the angle between longitudinal axes (A8, A9) of respective pins (302) and tangent line (T2).

With this configuration, using 2-pin rocker arm assembly (300), provides for achieving a ninety-degree contact angle, or thereabouts, for each of the pins retained by rocker arm assembly (300) when stabilizing when pinning into a planar region of the skull of the patient's head. In other words, pins (302) are perpendicularly pinned into the planar region of cranial bone in this arrangement shown in FIG. 6. Stated another way, rocker arm assembly (300) enables the surgeon or user to realize a perpendicular bone contact or penetration of pins (302) in rocker arm assembly (300) even though a planar region of cranial bone is pinned or used as a stabilizing location.

By way of further example, FIG. 8 shows portions of rocker arm assembly (300) of FIG. 6 without pins (302). In this example, rocker arm assembly (300), with arm (306) and bores (308), is configured for use in pinning into a planar region of cranial bone. Thus, when pinning into a planar region of cranial bone as shown in FIG. 8, the angle of contact is ninety degrees or about ninety degrees. More specifically, the contact angles (α7, α8) on each side of arm (306) are defined as the angle between longitudinal axes (A10, A11) of respective bores (308) and tangent line (T2).

As described above with respect to FIGS. 6 and 8, contact angle can be defined based on the orientation of bores (308) or the orientation of pins (302). In some instances where pins (302) are straight and bores (308) define concentric receptacles for receiving pins (302), the longitudinal axes defined by bores (308) and pins (302) will be the same. In this instance, the contact angles will be the same whether defined by the orientation of bores (308) or the orientation of pins (302). However, in some other examples, bores (308) may be omitted where pins (302) may be formed with, or as part of, arm (306). In such an example, the contact angle will be defined based on the orientation of the pins (302). Still in some other examples, bores (308) and pins (302) can be shaped or configured such that they do not have the same or a common longitudinal axis, and thus would have different contact angles as defined herein. By way of example only, and not limitation, this could be the case where pins (302) are angled or curved instead of straight. In view of the teachings herein, other configurations for rocker arm assembly (300) with pins (302) and bores (308), where pins (302) and bores (308) have different longitudinal axes, and thus different contact angles as defined herein, will be apparent to those of ordinary skill in the art. In application, where the contact angle defined by the bores and the contact angle defines by the pins differs, the contact angle as defined by the contact features or pins is a key factor when considering the stability of a patient stabilization as it is those contact features or pins that contact the patient's head.

In one version of exemplary 2-pin rocker arm assembly (300), arm (306) may be approximately 9 mm x 9 mm x 70 mm - 110 mm, with two perpendicular pin receptors or bores (308) at the ends of arm (306) at a distance of about 50 mm - 90 mm between each other. In another version of exemplary 2-pin rocker arm assembly (300), arm (306) is smaller, such that arm (306) is approximately 9 mm x 9 mm x 40 mm - 70 mm, with two perpendicular pin receptors or bores (308) at the ends of the arm at a distance of about 20 mm - 55 mm between each other. With this smaller configuration, 2-pin rocker arm assembly (300) may be configured for use with pediatric patent's or patent's having smaller head size.

In comparing rocker arm assembly (100) with rocker arm assembly (300), in each case respective pins (102, 302) define a longitudinal axis or centerline as shown in FIGS. 5 and 6. In some examples, the centerlines of each of the pair of pins intersect forming an angle, such as β1 in FIG. 5 for rocker arm assembly (100). This angle defines an angle of the arc, and may also be referred to herein as the radius or degree of curvature of the arm. With rocker arm assembly (100) as illustrated, angle β1 is forty degrees.

With rocker arm assembly (300) as illustrated in FIG. 6, the centerlines or longitudinal axes of pins (302) are parallel and thus do not intersect to define an angle. In other examples where the centerlines or longitudinal axes of pins (302) are close to parallel, but not exactly parallel, they intersect to define an angle as mentioned above. In such cases where these axes are close but not exactly parallel, the angle defined is less than forty degrees. Thus, with rocker arm assembly (300), the angle represented by β2 may be something that is equal to or greater than about zero degrees (as defined by β2 in FIG. 6) and less than forty degrees. It should be noted that herein a zero-degree angle for β2 is being defined as the case when the axes defined by pins (302) are parallel.

In this manner, a rocker arm assembly configured for pinning with a planar region of cranial bone can have a degree of curvature of the arm, defined by the angle formed by the longitudinal axes or centerlines of each of the pair of pins, that is greater than or equal to about zero degrees and less than forty degrees. For instance, when β2 is equal to zero degrees, arm (306) of rocker arm assembly (300) has a straight, non-curved shape. When β2 is greater than zero but less than about forty degrees, arm (106) of rocker arm assembly (100) can have a slight curvature. Thus, it should be understood that in some versions arm (306) is straight, while in some other versions arm (306) may have a curvature, but to a lesser degree than the curvature of rocker arm assemblies that are designed for pinning elliptically shaped cranial bone. Still in some other examples this range for the angle may be between about zero degrees and about thirty degrees, or between about zero degrees and about 20 degrees, or between about zero degrees and about 10 degrees. In view of the teachings herein, other such ranges will be apparent to those of ordinary skill in in the art.

In another comparison of rocker arm assembly (100) with rocker arm assembly (300), in each case respective bores (108, 308) define a longitudinal axis or centerline as shown in FIGS. 7 and 8. In some examples, the centerlines of each of the pair of bores (108, 308) intersect forming an angle, such as β3 in FIG. 7 for rocker arm assembly (100). This angle defines an angle of the arc, and may also be referred to herein as the radius or degree of curvature of the arm (106). With rocker arm assembly (100), angle β3 is forty degrees.

With rocker arm assembly (300) as illustrated in FIG. 8, the centerlines or longitudinal axes of bores (308) are parallel and thus do not intersect to define an angle. In other examples where the centerlines or longitudinal axes of bores (308) are close to parallel, but not exactly parallel, they intersect to define an angle as mentioned above. In such cases where these axes are close but not exactly parallel, the angle defined is less than forty degrees. Thus, with rocker arm assembly (300), the angle represented by β4 may be something that is equal to or greater than about zero degrees (as defined by β4 in FIG. 8) and less than forty degrees. It should be noted that herein a zero-degree angle for β4 is being defined as the case when the axes defined by bores (308) are parallel

In this manner, a rocker arm assembly configured for pinning with a planar region of cranial bone can have a degree of curvature of the arm, defined by the angle formed by the longitudinal axes or centerlines of each of the pair of bores, that is greater than or equal to about zero degrees and less than forty degrees. For instance, when β4 is equal to zero degrees, arm (306) of rocker arm assembly (300) has a straight, non-curved shape. When β4 is greater than zero but less than forty degrees, arm (106) of rocker arm assembly (100) can have a slight curvature. Thus, it should be understood that in some versions arm (306) is straight, while in some other versions arm (106) may have a curvature, but to a lesser degree than the curvature of rocker arm assemblies designed for pinning elliptically shaped cranial bone. Still in some other examples this range for the angle may be between about zero degrees and about thirty degrees, or between about zero degrees and about 20 degrees, or between about zero degrees and about 10 degrees. In view of the teachings herein, other such ranges will be apparent to those of ordinary skill in in the art.

As mentioned above, rocker arm assemblies, like rocker arm assembly (100) are configured for use with elliptically shaped cranial bone. Such rocker arm assemblies comprise an angle-defined by the longitudinal axes or centerlines of either the pair of bores within the arm of the rocker arm, or the pair of pins within the arm of the rocker arm-of forty degrees or more. When such rocker arm assemblies are used to pin into planar regions of cranial bone, the result can be contact angles that deviate significantly from a perpendicular penetration angle such that a stable and/or rigid stabilization may be compromised. However, as shown and described above with respect to rocker arm assembly (300), using a stabilization configuration where the angle-defined by the longitudinal axes or centerlines of either the pair of bores within the arm of the rocker arm, or the pair of pins within the arm of the rocker arm-is less than forty degrees provides for the ability to achieve a stable and/or rigid stabilization of a patient in procedures that involve pinning the head in planar regions of cranial bone as the contact angle can be maintained at or close to ninety degrees.

### III. Miscellaneous

The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims

Having shown and described various embodiments of the present invention, further adaptations of the methods and systems described herein may be accomplished by appropriate modifications by one of ordinary skill in the art without departing from the scope of the claims. Several of such potential modifications have been mentioned, and others will be apparent to those skilled in the art. Accordingly, the scope of the present invention should be considered in terms of the following claims and is understood not to be limited to the details of structure and operation shown and described in the specification and drawings.

## Claims

1. A head fixation device to stabilize a head of a patient, the device comprising:
(a) a rocker arm assembly (100, 300) comprising an arm (106, 306), wherein the rocker arm assembly defines a rotational axis about which the arm is rotatable;
(b) two contact features (102, 302) retained by the rocker arm assembly or two bores configured to each selectively retain a contact feature (102, 302) for contacting the head of the patient, wherein the contact features are configured to contact the head of the patient, wherein each of the contact features or each of the bores defines a longitudinal axis that extends through a distal end of the contact feature configured to contact the head of the patient or through the bore, and
(c) a pin holder (104) opposite the rocker arm assembly, wherein the pin holder includes a third contact feature or bore configured to contact the head of the patient,
**characterized in that** the contact features or bores form an isosceles triangle and an angle defined by the two longitudinal axes of the contact features or bores retained by the rocker arm assembly is less than about 30 degrees.

2. The device of claim 1, wherein the angle defined between the two longitudinal axes is determined by a configuration where the contact features defining the two longitudinal axes or two of the contact features configured to be retained within the respective bores each define a contact angle with a substantially planar portion of cranial bone of the patient, wherein the contact angle is about 90 degrees.

3. The device of claim 1, wherein at least two of the two or more contact features (102, 302) are configured to contact a substantially planar portion of the head of the patient.

4. The device of claim 1, wherein the contact features (102, 302) are selectively retained by the arm of the rocker arm assembly.

5. The device of claim 1, wherein the contact features or the bores defining the two longitudinal axes each define a contact angle with a substantially planar portion of cranial bone of the patient, wherein the contact angle is about 90 degrees, and wherein the contact angle represents a penetration angle.

6. The device of claim 1, wherein the arm (106, 306) of the rocker arm assembly is non-curved.

7. The device of claim 1, wherein the arm comprises the two or more bores (108, 308) each configured to retain one of the contact features.

8. The device of claim 1, wherein the arm (106, 306) of the rocker arm assembly defines a fixed shape.

9. The device of claim 1, wherein the relative distance of the contact features (102, 302) to each other is fixed.

10. The device of one of claims 1-9, wherein the angle defined by the longitudinal axes is less than about 20 degrees.

11. The device of one of claims 1-9, wherein the angle defined by the longitudinal axes is less than about 10 degrees.

12. The device of one of claims 1-9, wherein the angle defined by the longitudinal axes is preferably about 0 degrees.

## Patentansprüche

1. Kopffixierungsvorrichtung zur Stabilisierung des Kopfes eines Patienten, wobei die Vorrichtung Folgendes umfasst:
a) eine Kipphebelanordnung (100, 300) mit einem Arm (106, 306), wobei die Kipphebelanordnung eine Drehachse definiert, um die der Arm drehbar ist;
b) zwei Berührungselemente (102, 302), die durch die Kipphebelanordnung gehalten werden, oder zwei Aushöhlungen, die so konfiguriert sind, dass sie jeweils selektiv ein Berührungselement (102, 302) halten, um mit dem Kopf des Patienten in Kontakt zu kommen, wobei die Berührungselemente so konfiguriert sind, dass sie mit dem Kopf des Patienten in Kontakt kommen, wobei jedes der Berührungselemente oder jede der Aushöhlungen eine Längsachse definiert, die sich durch ein distales Ende des Berührungselements, das so konfiguriert ist, dass es mit dem Kopf des Patienten in Kontakt kommt, oder durch die Aushöhlung erstreckt, und
c) einen Stifthalter (104) auf der gegenüberliegenden Seite der Kipphebelanordnung, wobei der Stifthalter ein drittes Berührungselement oder eine dritte Aushöhlung umfasst, die so konfiguriert sind, dass sie mit dem Kopf des Patienten in Kontakt kommt,
d) **dadurch gekennzeichnet, dass** die Berührungselemente oder Aushöhlungen ein gleichschenkliges Dreieck bilden und ein Winkel, der durch die beiden Längsachsen der Berührungselemente oder Aushöhlungen definiert wird, die von der Kipphebelanordnung gehalten werden, kleiner als etwa 30 Grad ist.

2. Vorrichtung nach Anspruch 1, wobei der zwischen den beiden Längsachsen definierte Winkel durch eine Konfiguration bestimmt wird, bei der die die beiden Längsachsen definierenden Berührungselemente oder zwei der Berührungselemente, die so konfiguriert sind, dass sie in den jeweiligen Aushöhlungen gehalten werden, jeweils einen Berührungswinkel mit einem im Wesentlichen ebenen Abschnitt des Schädelknochens des Patienten definieren, wobei der Berührungswinkel etwa 90 Grad beträgt.

3. Vorrichtung nach Anspruch 1, wobei mindestens zwei der zwei oder mehr Berührungselemente (102, 302) so konfiguriert sind, dass sie mit einem im Wesentlichen ebenen Abschnitt des Kopfes des Patienten in Kontakt kommen.

4. Vorrichtung nach Anspruch 1, wobei die Berührungselemente (102, 302) selektiv durch den Arm der Kipphebelanordnung gehalten werden.

5. Vorrichtung nach Anspruch 1, wobei die Berührungselemente oder die die beiden Längsachsen definierenden Aushöhlungen jeweils einen Berührungswinkel mit einem im Wesentlichen ebenen Teil des Schädelknochens des Patienten definieren, wobei der Berührungswinkel etwa 90 Grad beträgt und wobei der Berührungswinkel einen Eindringwinkel darstellt.

6. Vorrichtung nach Anspruch 1, wobei der Arm (106, 306) der Kipphebelanordnung nicht gekrümmt ist.

7. Vorrichtung nach Anspruch 1, wobei der Arm zwei oder mehr Aushöhlungen (108, 308) umfasst, die jeweils so konfiguriert sind, dass sie eines der Berührungselemente halten.

8. Vorrichtung nach Anspruch 1, wobei der Arm (106, 306) der Kipphebelanordnung eine feste Form definiert.

9. Vorrichtung nach Anspruch 1, wobei der relative Abstand der Berührungselemente (102, 302) zueinander fest ist.

10. Vorrichtung nach einem der Ansprüche 1 bis 9, wobei der durch die Längsachsen definierte Winkel weniger als etwa 20 Grad beträgt.

11. Vorrichtung nach einem der Ansprüche 1 bis 9, wobei der durch die Längsachsen definierte Winkel weniger als etwa 10 Grad beträgt.

12. Vorrichtung nach einem der Ansprüche 1 bis 9, wobei der durch die Längsachsen definierte Winkel vorzugsweise etwa 0 Grad beträgt.

## Revendications

1. Dispositif de fixation de tête pour stabiliser la tête d'un patient, le dispositif comprenant :
(a) un ensemble culbuteur (100, 300) comprenant un bras (106, 306), dans lequel l'ensemble culbuteur définit un axe de rotation autour duquel le bras peut tourner ;
(b) deux éléments de contact (102, 302) retenus par l'ensemble culbuteur ou deux alésages configurés pour retenir chacun sélectivement un élément de contact (102, 302) pour être en contact avec la tête du patient, dans lequel les éléments de contact sont configurés pour être en contact avec la tête du patient, dans lequel chacun des éléments de contact ou chacun des alésages définit un axe longitudinal qui s'étend à travers une extrémité distale de l'élément de contact configuré pour être en contact avec la tête du patient ou à travers l'alésage, et
(c) un support de broche (104) opposé à l'ensemble culbuteur, dans lequel le support de broche comprend un troisième élément de contact ou alésage configuré pour être en contact avec la tête du patient,
**caractérisé en ce que** les éléments de contact ou alésages forment un triangle isocèle et un angle défini par les deux axes longitudinaux des éléments de contact ou alésages retenus par l'ensemble culbuteur est inférieur à environ 30 degrés.

2. Dispositif selon la revendication 1, dans lequel l'angle défini entre les deux axes longitudinaux est déterminé par une configuration dans laquelle les éléments de contact définissant les deux axes longitudinaux ou deux des éléments de contact configurés pour être retenus dans les alésages respectifs définissent chacun un angle de contact avec une partie sensiblement plane de l'os crânien du patient, dans lequel l'angle de contact est d'environ 90 degrés.

3. Dispositif selon la revendication 1, dans lequel au moins deux des deux éléments de contact (102, 302) ou plus sont configurés pour être en contact avec une partie sensiblement plane de la tête du patient.

4. Dispositif selon la revendication 1, dans lequel les éléments de contact (102, 302) sont sélectivement retenus par le bras de l'ensemble culbuteur.

5. Dispositif selon la revendication 1, dans lequel les éléments de contact ou les alésages définissant les deux axes longitudinaux définissent chacun un angle de contact avec une partie sensiblement plane de l'os crânien du patient, dans lequel l'angle de contact est d'environ 90 degrés, et dans lequel l'angle de contact représente un angle de pénétration.

6. Dispositif selon la revendication 1, dans lequel le bras (106, 306) de l'ensemble culbuteur n'est pas courbé.

7. Dispositif selon la revendication 1, dans lequel le bras comprend les deux alésages (108, 308) ou plus chacun configurés pour retenir l'un des éléments de contact.

8. Dispositif selon la revendication 1, dans lequel le bras (106, 306) de l'ensemble culbuteur définit une forme fixe.

9. Dispositif selon la revendication 1, dans lequel la distance relative des éléments de contact (102, 302) entre eux est fixe.

10. Dispositif selon l'une des revendications 1 à 9, dans lequel l'angle défini par les axes longitudinaux est inférieur à environ 20 degrés.

11. Dispositif selon l'une des revendications 1 à 9, dans lequel l'angle défini par les axes longitudinaux est inférieur à environ 10 degrés.

12. Dispositif selon l'une des revendications 1 à 9, dans lequel l'angle défini par les axes longitudinaux est de préférence d'environ 0 degré.
